# EUROPEAN PATENT APPLICATION

(11) **EP 2 044 849 A1**
(43) Date of publication of application: **08.04.2009**
(21) Application number: 07019018.6
(22) Date of filing: 27.09.2007
(51) Int. Cl.: A23L 1/212, A23L 1/30, A23L 1/302, A23L 2/02, A23L 2/52, A61K 8/97, A61K 31/05, A61K 36/47

(54) **Process for the manufacure of an amla composition**

(71) Applicant: Scindia AG, 6060 Sarnen (CH); Innovative Naturals Inc., Paschim Vihar Dehli 110063 (IN)
(72) Inventor: Krishan, Gopi, Delhi 110063 (IN); Buecker, Rolf Dieter, 87781 Ungerhausen (DE)
(74) Representative: Grimm, Siegfried

(57) **Abstract**

A process for the manufacture of an amla composition is disclosed, comprising the steps of: (a) providing amla extract starting material; (b) adding several volumes of amla juice to the starting material; (c) subjecting the mixture to a concentration process; and (d) adding further amla juice to the mixture, repeating the concentration process. In addition, an amla comprising composition is described.

## Description

The invention discloses a process for producing a fortified amla composition, as well as a fortified amla composition.

Amla, also called Indian gooseberry *(Emblica officinalis),* is a tree belonging to the Euphorbiaceae family, native to the Indian costal areas and plains and Kashmir. Especially its edible fruit has found an extensive use in dried and fresh form in ancient Ayurvedic medicine. The promotion of longevity, strengthening the lungs, helping to fight chronic lung problems as well as upper respiratory infections is attributed to the fruit.

The amla fruit has a high content of vitamin C, allegedly being up to 720 mg of per 100 g of fresh fruit pulp, or up to 900 mg per 100 g of pressed juice. Furthermore, amla also contains a number of polyphenols (tannins and flavonoids). Vitamin C and polyphenols serve upon ingestion as anti-oxidants.

Apart from human consumption, amla extracts are used in inks, dyes, shampoos and hair oils.

The extract is traditionally produced by adding 5 times the volume of fresh amla juice to dried amla fruits. The mixture is left at room temperature until the liquid has evaporated. The procedure may be repeated a number of times, each time adding juice to the remaining and letting the liquid evaporate. Said repeats lead to a so-called "fortified" product, resulting in a high quality product with regard to the content of Vitamin C and polyphenols. Because the process is done at room temperature and atmospherical pressure, the evaporation takes time and thus the process is industrially not applicable.

Due to the widespread use of amla extracts in commercial goods, a quicker, industrialized process was developed, wherein the dried amla fruits are extracted with 95% of alcohol in a ratio of 1 kg of fruit to 2 kg of 95% alcohol. An extract is obtained by separating the mixture and submitting it to vacuum extraction. Although this process is quick, the product is not as rich in the overall concentrations of polyphenols and/or vitamin C as the one produced by the traditional process.

Therefore, there is a need for an improved process which on one hand is apt for use at industrial scale, and at the same time provides a product being comparable in quality to the traditional process.

Hence, it is a general object of the invention to provide a manufacturing process for a fortified amla composition which can be operated at industrial scale and generates a product rich in anti-oxidants.

In another aspect, the present invention relates to fortified amla compositions with a high overall concentration of vitamin C and polyphenols.

The process of the present invention comprises the steps of:
a) providing amla extract starting material;
b) adding amla juice to the starting material;
c) subjecting the mixture to a concentration process under reduced pressure;
d) adding further amla juice to the obtained mixture and
e) repeating steps c) and d).

The amla extract starting material is preferably a solvent extract of the amla fruit, preferably in dried form. Said extract may stem from any solvent, but preferably a 5:1 or 6:1 extract of dried amla fruit in 90 to 95% volume % alcohol is used (i.e. five or six kilograms of dried fruit yield one kg of final alcohol extract). The manufacture of such amla extracts is known in the field.

Amla juice is added to the amla extract starting material, preferably, fresh amla juice is used. Most preferably, the amla juice is filtered and/or clarified.

The ratio of amla extract starting material and added liquid may vary. Preferably, if the starting material stems from 1 kg of extract, at least 25 kg of juice are added during the entire process.

Fortification is performed by subjecting the mixture to a concentration process under reduced pressure. The concentration process is either a one step process or a multi-step process, e.g. comprising a rotary vacuum concentration process and/or a thin film concentration process. For example, a Buchi-type rotary vacuum evaporator having a bath temperature below 90°C under almost complete vacuum (e.g. 20 mbar) may be used.

The mixture is preferably concentrated to at least half its initial volume

After concentrating the mixture, further amla juice is added to the obtained mixture. Preferably, fresh amla juice is used, most preferably, the amla juice is filtered and/or clarified. Concentration step c) is then repeated.

Depending on the concentration process used, the addition of juice to the mixture may be batch like or in a continuous manner. For example, in a rotary evaporator, the juice may be present in a separate flask, from which liquid is added continuously to the mixture.

Step e) can be repeated a number of times, preferably at least five times, more preferably at least 25 times. The initial can be fortified with 50 times or up to 100 times with fresh juice and can be similarly fortified in rotary vacuum concentration and/or in a thin film concentration process. The fortification may be repeated as many times until a desired polyphenol/vitamin C concentration is attained.

In a further aspect, the invention encompasses a composition obtainable or obtained by the process described herein. Said composition is preferably at least 25 times fortified when compared to the starting material.

The process results in a fortified amla composition, typically comprising at least 45 % natural polyphenols and at least 5% natural vitamin C on w/w (weight by weight) basis.

Unless otherwise specified, all percentages are indicated as weight percent.

Further, the water content of the final amla composition is typically in the range of 4%-7%, preferably 4%.

The fortified product may e.g. be vacuum dried and used for the formulation of a multiplicity of products.

The composition may be used as any conventional amla extract, but preferably as an additive for foods, beverages, cosmetics and/or pharmaceuticals.

The inventive composition is e.g. suitable for augmenting the anti-oxidant potential of confectionary food products such as chocolates or candies and of health drinks such as barley water. By adding the inventive amla composition a content of more than 45% polyphenols and a vitamin C content of more than 5% may be achieved without addition of synthetic ascorbic acid (vitamin C).

The fortified amla composition with the desired polyphenol/vitamin C ratio/concentration may be mixed with sugar and/or misri to achieve a palatable product for consumption. In the scope of the present invention, the term "misri" refers to a refined product obtained from white sugar (i.e. sucrose). It is obtainable e.g. by mixing a super saturated sucrose solution with 1.5% low fat milk and crystallizing the final product by using cotton threads.

The composition may be eaten as such or mixed with other sugar or non-sugary food products to impart health benefits to the food products currently in use. Through addition of the inventive composition presently available sugary food products may be converted to a class of super foods rich in anti-oxidants (polyphenols) and naturally occurring vitamin C. Said amla composition is capable of preventing insulin resistance, overweight, obesity and/or metabolic disorders caused by consumption of present day available foods

In a further aspect, the invention encompasses beverages, food or pharmaceuticals containing the composition and optionally also comprise sugar and/or misri.

In a further aspect, the composition may be used as a medicament.

Further to the above mentioned uses, the composition may be used for the manufacture of a medicament for the treatment, prevention, delay of progression of a disorder selected from the group of insulin resistance, overweight, obesity and metabolic disorders.

### Modes for Carrying Out the Invention

The invention can be carried out using standard Buchi rotary vacuum evaporator size 1 Litre/ 2 Litre/ 5 Litre/ 20 Litre or can be carried out in rotary vacuum evaporators of similar designs, e.g. equipments made in Baroda (India). Typically, the water bath temperature is around 90 °C, the rotating flask speed adjusted to ensure appropriate evaporation and almost complete vacuum is adjusted.

While there are shown and described presently preferred embodiments of the invention, it is to be distinctly understood that the invention is not limited thereto but may be otherwise variously embodied and practiced within the scope of the following claims.

## Claims

1. A process for the manufacture of an amla composition comprising the steps of:
a) providing amla extract starting material;
b) adding amla juice to the starting material;
c) subjecting the mixture to a concentration process under reduced pressure;
d) adding further amla juice to the obtained mixture and
e) repeating steps c) and d).

2. The process according to claim 1, wherein the starting material of step a) contains or essentially is a solvent extract of the amla fruit.

3. The process of claim 1 or 2, wherein the starting material is a 5:1 amla extract in 90 to 95% alcohol.

4. The process of anyone of the preceding claims, wherein the amla juice used in step b) and d) is filtered and/or clarified.

5. The process of anyone of the preceding claims, wherein the concentration process of step c) is a one-step or a multi-step process comprising a rotary vacuum concentration process and/or a thin film concentration process.

6. The process of anyone of the preceding claims, wherein step e) is repeated at least five times, preferably at least 25 times.

7. A composition obtained by the process according to claims 1 to 6; in particular wherein the starting material is at least 25 times fortified.

8. The composition of claim 7, comprising at least 45 % natural poly phenols and at least 5% natural Vitamin C on weight/weight basis.

9. Use of a composition of anyone of claims 7 to 8 as food additive, beverage additive, an additive for cosmetics and/or pharmaceutical additive.

10. A beverage, food or pharmaceutical containing a composition of claim 7 or 8 and optionally further comprising sugar and/or misri.

11. The composition of claim 7 or 8 as medicament.

12. Use of a composition according to claim 7 or 8 for the manufacture of a medicament for the treatment, prevention, delay of progression of a disorder selected from the group of insulin resistance, overweight, obesity and metabolic disorders.
